(19) Europäisches Patentamt
European Patent Office
Office européen des brevets

(11) Publication number: 0 351 214
A1

(12) # EUROPEAN PATENT APPLICATION

(21) Application number: 89307096.1

(22) Date of filing: 13.07.89

(51) Int. Cl.[5]: C 07 C 259/06
A 61 K 31/16

(30) Priority: 14.07.88 EP 88306446
20.10.88 GB 8824560
02.11.88 GB 8825669

(43) Date of publication of application:
17.01.90 Bulletin 90/03

(84) Designated Contracting States:
AT BE CH DE ES FR GB GR IT LI LU NL SE

(71) Applicant: THE WELLCOME FOUNDATION LIMITED
183-193 Euston Road
London NW1 2BP (GB)

(72) Inventor: Jackson, William Paul
The Wellcome Research Laboratories Langley Court
Beckenham Kent BR 3 3BS (GB)

Salmon, John Anthony
The Wellcome Research Laboratories Langley Court
Beckenham Kent BR 3 3BS (GB)

(74) Representative: Garrett, Michael et al
The Wellcome Foundation Limited Group Patents and
Agreements Langley Court
Beckenham Kent BR3 3BS (GB)

(54) **Anti-inflammatory aryl derivatives.**

(57) The present invention is concerned with (E)-N-{1(S)-methyl-3-[3-(4-fluorophenoxy)phenyl]prop-2-en-1-yl}acetohydroxamic acid and salts thereof, pharmaceutical formulations containing them, processes for their preparation, and uses for them in medicine and other applications.

EP 0 351 214 A1

**Description**

## ANTI-INFLAMMATORY ARYL DERIVATIVES

The present invention is concerned with an acetohydroxamic acid aryl derivative and salts thereof, with pharmaceutical formulations containing them, with processes for their preparation, and with their use in medicine and other applications.

A class of agents defined in European Patent 55418 are described therein as dual inhibitors of the lipoxygenase and cyclo-oxygenase enzymes of the mammalian arachidonic acid metabolism and were found to exhibit anti-inflammatory and related activities. Other compounds which have been described as lipoxygenase and/or cyclo-oxygenase inhibitors include certain naphthyloxy derivatives, for example, as described in U.S. Patent 3,740,437 or in Proc. Ann. Symp. Inst. Basic Med. Sci., Royal College of Surgeons of England, October 1982, pp. 263-274. Compounds described in the latter reference include the compound known as nafazatrom.

European Patent Application No. 86301895 discloses a class of compounds which are inhibitors of the lipoxygenase and/or cyclo-oxygenase enzymes. Within the class of compounds described in this application was identified a sub-class of compounds having exceptional activity with regard to the inhibition of lipoxygenase and/or cyclo-oxygenase. This sub-class of compounds is disclosed in European Patent Application No 88306446. We have now found that within the sub-class of compounds described in the later application, there is a particular compound in a particular enantiomeric form which has outstanding properties as an inhibitor of lipoxygenase and/or cyclo-oxygenase which render it particularly useful for certain medical and non-medical applications.

According to the later European application, there is provided a compound of formula (I)

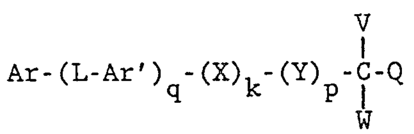

(I)

wherein

q is 1, k is 0, and p is 1;

Ar is phenyl optionally substituted by one or more substituents independently selected from $C_{1-4}$ alkyl (which may be substituted by one or more halogen atoms) and halogen;

L is -0-;

Ar′ is 1,3- or 1,4-phenylene;

Y is (E)-CH=CH-;

V is hydrogen or $C_{1-4}$ alkyl;

W is $C_{1-4}$ alkyl; and

Q is a moiety of formula

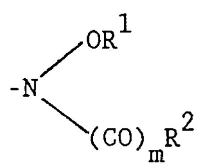

wherein m is 1, $R^1$ is hydrogen, and $R^2$ is hydrogen or $C_{1-4}$ alkyl;

and salts thereof.

As indicated, we have now found that within the sub-class of compounds described in European Patent Application No 88306446, there is a particular compound in a particular enantiomeric form which has outstanding properties as an inhibitor of lipoxygenase and/or cyclo-oxygenase, particularly in respect of its activity and duration of action.

According to the present invention, therefore, there is provided

(E)-N-{1(S)-Methyl-3-[3-(4-fluorophenoxy)phenyl]prop-2-en1-yl}-acetohydroxamic acid;

and salts thereof.

The invention further includes bioprecursors or "pro-drugs" of the compound of the invention, that is, compounds which are converted in vivo to the compound of the invention or to one of its physiologically acceptable salts.

According to further aspects of the invention, there are provided pharmaceutical formulations containing the compound of the invention and/or one of its physiologically acceptable salts, processes for the preparation of the compound and its salts, and uses for them in medicine and other applications.

For use in medicine, suitable salts of the compound of the invention are those which are physiologically acceptable. However, non-physiologically acceptable salts are included within the ambit of the present invention, either for use in non-medical applications such as further described hereinbelow, or for use as intermediates in the preparation of the compound of the invention and its physiologically acceptable salts.

Acid addition salts according to the invention include the acetate, adipate, alginate, aspartate, benzoate, benzenesulphonate, bisulphate, butyrate, citrate, camphorate, camphorsulphonate, cyclopentanepropionate, digluconate, dodecylsulphate, ethanesulphonate, fumarate, glucoheptanoate, glycerophosphate, hemisulphate, heptanoate, hexanoate, hydrochloride, hydrobromide, hydroiodide, 2-hydroxyethanesulphonate, lactate, maleate, methanesulphonate, 2-naphthalenesulphonate, nicotinate, oxalate, palmonate, pectinate, persulphate, 3-phenylpropionate, picrate, pivalate, proprionate, succinate, tartrate, thiocyanate, tosylate and undeconoate.

Base salts according to the invention include ammonium salts, alkali metal salts such as those of sodium and potassium, alkaline earth metal salts such as those of calcium and magnesium, salts with organic bases such as dicyclohexylamine and N-methyl-D-glucamine, and salts with amino acids, such as arginine and lysine.

Quaternary ammonium salts according to the invention may be prepared when a nitrogen-containing group is present, for example, by reaction with lower alkyl halides, such as methyl, ethyl, propyl, and butyl chlorides, bromides and iodides, with dialkyl sulphates, with long chain halides such as decyl, lauryl, myristyl and stearyl chlorides, bromides and iodides, and with aralkyl halides such as benzyl and phenethyl bromides.

Subject to any limitations expressed or implied herein, the present invention also provides the compound of formula (I), or any physiologically acceptable salt thereof, for use as an inhibitor of the lipoxygenase and/or cyclo-oxygenase enzymes of the mammalian arachidonic acid metabolism, to methods of inhibition of such enzyme(s) by administration to a mammal of a lipoxygenase and/or cyclo-oxygenase (as appropriate) inhibiting amount of any such compound or salt, and to the use of any such compound or salt in the manufacture of lipoxygenase and/or cyclo-oxygenase inhibiting (as appropriate) agents.

Further, and also subject to any limitations expressed herein, the present invention also provides the compound of formula (I), or any physiologically acceptable salt thereof, for use as a medical therapeutic and/or prophylactic agent, to methods of medical therapeutic and/or prophylactic treatment by administration to a mammal of a medically therapeutic and/or prophylactic (as appropriate) effective amount of any such compound or salt, and to the use of any such compound or salt in the manufacture of medically therapeutic and/or prophylactic (as appropriate) agents. The kinds of medical therapy and prophylaxis pertinent to the foregoing are elaborated by way of example in the following paragraphs, but are not intended to be construed as in any way limiting the scope of these aspects of the invention.

By virtue of their lipoxygenase inhibitory properties, said compound and salts find application in the treatment and/or prophylaxis of any condition where a lipoxygenase inhibitor is indicated, especially spasmogenic and allergic conditions and tumours.

By virtue of their cyclo-oxygenase inhibitory properties, said compound and salts find application in the treatment and/or prophylaxis of any condition where a cyclo-oxygenase inhibitor is indicated, especially pyresis and pain.

By virtue of both their lipoxygenase and cyclo-oxygenase inhibitory properties, said compound and salts find application in the treatment and/or prophylaxis of any condition where a dual lipoxygenase/cyclo-oxygenase inhibitor is indicated, especially any condition involving blood platelet aggregation or inflammation. In the case of inflammation, the compounds and salts of the invention are particularly suited to the treatment and/or prophylaxis of conditions associated with the infiltration of leucocytes into inflamed tissue.

In determining whether a lipoxygenase, cyclo-oxygenase or dual lipoxygenase/cyclo-oxygenase inhibitor is indicated, of course inter alia, the particular condition in question and its severity must be taken into consideration and the determination is ultimately at the discretion of the attendant physician.

Examples of the aforesaid spasmogenic conditions are those involving smooth muscle tissue, especially airway smooth muscle constriction such as intrinsic asthma (including intrinsic or idiopathic bronchial asthma and cardiac asthma), bronchitis and arterial smooth muscle constriction such as coronary spasm (including that associated with myocardial infarction, which may or may not lead to left ventricular failure resulting in cardiac asthma) and cerebral spasm or 'stroke'. Other examples include bowel disease caused by abnormal colonic muscular contraction such as the condition termed 'irritable bowel syndrome', 'spastic colon' and 'mucous colitis'.

Examples of the aforesaid allergic conditions are extrinsic asthma (from which it will be appreciated that the compounds and salts of the invention are particularly favourable as anti-asthmatic agents), allergic skin diseases having a total or partial allergic origin, such as eczema, allergic bowel diseases (including coeliac disease), allergic eye conditions, such as hayfever (which may additionally or alternatively affect the upper respiratory tract), and allergic conjunctivitis.

Examples of the aforesaid tumours are skin neoplasms, both benign and malignant.

Examples of the aforesaid pyretic and painful conditions include fever associated with infections, trauma and injury, malignant disease, and diseases affecting the immune system (including anto-immune diseases).

Examples of the aforesaid condition involving blood platelet aggregation are those resulting from thrombosis, including 'strokes' having a total or partial thrombotic origin, coronary thrombosis, phlebitis and phlebothrombosis (the latter two conditions also possible being associated with inflammation).

Examples of the aforesaid conditions involving inflammation are inflammatory conditions of the lung, joints, eye, bowel, skin and heart.

Inflammatory lung conditions which may be treated and/or prevented include asthma and bronchitis (vide supra) and cystic fibrosis (which may additionally or alternatively involve the bowel or other tissue(s)).

Inflammatory joint conditions which may be treated and/or prevented include rheumatoid arthritis,

rheumatoid spondylitis, osteoarthritis, gouty arthritis and other arthritic conditions.

Inflammatory eye conditions which may be treated and/or prevented include uveitis (including iritis) and conjunctivitis (vide supra).

Inflammatory bowel conditions which may be treated and/or prevented include Crohn's disease, ulcerative colitis and distal proctitis.

Inflammatory skin diseases which may be treated and/or prevented include those associated with cell proliferation, such as psoriasis and eczema (vide supra) and dermatitis (whether or not of allergic origin).

Inflammatory conditions of the heart which may be treated and/or prevented include coronary infarct damage.

Other inflammatory conditions which may be treated and/or prevented include tissue necrosis in chronic inflammation and tissue rejection following transplant surgery.

It is also believed that the compound of formula (I) and its physiologically acceptable salts are effective agents in the prophylaxis and/or treatment of (i) atherosclerosis, (ii) bacterial and fungal infections and (iii) dysmenorrhoea, these applications being further aspects of the present invention.

It is known from the literature that some compounds which are cyclo-oxygenase and/or lipoxygenase inhibitors can delay the decay of cut plant matter. Thus it is believed that by virtue of their enzyme inhibitory effects, the compound of formula (I) and its salts are also useful for controlling the processes of growth and decay in plants. Therefore the present invention also provides the compound of formula (I) and its salts for use in a method of regulating the growth of, or delaying senescence in, vegetable matter by application to said matter of an effective amount of the compound of formula (I) or a salt thereof.

The term "senescence" refers to the process whereby plant matter decays, especially after being picked, cut or otherwise removed from its normal growing environment. Vegetable matter includes trees, shrubs, flowers, edible vegetables and other food crops.

The above method is particularly applicable to flowers intended for decorative or display purposes such as carnations, cyrsanthemums. daisies, begonias, etc. These include perennial annual and biannual flowers, for example, those that grow from bulbs (e.g. dahlias) or from seed (e.g. marigolds). The method is also especially suitable for use with decorative shrubs and trees, for example, those which are displayed when cut, such as Christmas trees.

The compound of formula (I) and its salts may also be used for the preservation of picked fruits.

For medical use, the amount required of a compound of formula (I) of a physiologically acceptable salt thereof (hereinafter referred to as the active ingredient) to achieve a therapeutic effect will, of course, vary with the particular compound, the route of administration, the mammal under treatment, and the particular disorder or disease being treated. A suitable dose of the compound of formula (I) or of a physiologically acceptable salt thereof for a mammal suffering from, or likely to suffer from, any condition as described hereinbefore is 0.1μg-500mg of base per kilogram bodyweight. In the case of systemic administration, the dose is typically in the range 0.5 to 500mg of base per kilogram bodyweight, the most preferred dosage being 0.5 to 50mg/kg bodyweight, for example, 5 to 25mg/kg, administered two or three times daily. In the case of topical administration, e.g. to the skin or eye, a suitable dose is in the range 0.1ng-100μg of base per kilogram, typically about 0.1μg/kg.

In the case of oral dosing for the treatment or prophylaxis of airway smooth muscle constriction, or asthma or bronchitis in general, due to any cause, a suitable dose of the compound of formula (I) or of a physiologically acceptable salt thereof may be as specified in the preceding paragraph, but most preferably is from 1mg to 10mg of base per kilogram bodyweight, the most preferred dosage being from 1mg to 5mg/kg bodyweight, for example, from 1 to 2 mg/kg. In the case of pulmonary administration for the latter indications, the dose is typically in the range 2μg to 100mg/kg for example, from 20μg to 0.5mg/kg, especially from 0.1 to 0.5 mg/kg.

While it is possible for the active ingredient to be administered alone, it is preferable to present it as a pharmaceutical formulation comprising the compound of formula (I) or a pharmacologically acceptable salt thereof and a physiologically acceptable carrier therefor. Such formulations constitute a further feature of the present invention. Generally, the active ingredient comprises from 0.1% to 99.9% by weight of the formulation. Typically, unit doses of a formulation according to the invention contain from 0.1mg to 1g of the active ingredient. For topical administration, the active ingredient preferably constitutes from 1% to 2% by weight of the formulation, but the active ingredient may constitute as much as 10% w/w. Formulations suitable for nasal or buccal administration (such as the self-propelling powder dispensing formulations described below), typically comprise from 0.1 to 20% w/w, for example, 2% w/w of active ingredient.

The formulations of the present invention, both for veterinary and human medical use, comprise an active ingredient in association with a pharmaceutically acceptable carrier therefor and optionally other therapeutic ingredient(s). The carrier(s) must be acceptable in the sense of being compatible with the other ingredients of the formulation and must not be detrimental to the recipient.

Formulations according to the invention include those in a form suitable for oral, pulmonary, ophthalmic, rectal, parenteral (including subcutaneous, intramuscular and intravenous), intra-articular, topical, nasal, or buccal administration.

The formulations of the invention may conveniently be presented in unit dosage form and may be prepared by any method well known in the art of pharmacy. All such methods include the step of bringing the active ingredient into association with a carrier which constitutes one or more accessory ingredients. In general, the formulations are prepared by uniformly and intimately bringing the active ingredient into association with a

liquid carrier or a finely divided solid carrier, or both, and then, if desired, shaping the product into the required form.

Formulations according to the present invention which are suitable for oral administration may be in the form of discrete units such as capsules, cachets, tablets, or lozenges, each containing a predetermined amount of the active ingredient; in the form of a powder or granules; in the form of a solution or a suspension in an aqueous or non-aqueous liquid; or in the form of an oil-in-water or water-in-oil emulsion. The active ingredient may also be in the form of a bolus, electuary, or paste.

A tablet may be made by compressing or moulding the active ingredient, optionally with one or more accessory ingredients. Compressed tablets may be prepared by compressing, in a suitable machine, the active ingredient in a free-flowing form such as a powder or granules, optionally mixed with a binder, lubricant, inert diluent, and/or surface active or dispersing agent. Moulded tablets may be made by moulding, in a suitable machine, a mixture of the powdered active ingredient and a suitable carrier moistened with an inert liquid diluent.

Formulations for rectal administration may be in the form of a suppository incorporating the active ingredient and a carrier such as cocoa butter, or in the form of an enema.

Formulations suitable for parenteral administration typically comprise a sterile aqueous preparation of the active ingredient which is preferably isotonic with the blood of the recipient.

Formulations suitable for intra-articular administration may be in the form of a sterile aqueous preparation of the active ingredient, which latter may be in microcrystalline form, for example, an aqueous microcrystalline suspension. Liposomal formulations and biodegradable polymer systems may also be used to present the active ingredient for both intra-articular and ophthalmic administration.

Formulations suitable for topical administration include liquid and semi-liquid preparations such as liniments, lotions and applications; oil-in-water and water-in-oil emulsions such as creams, ointments and pastes; and solutions and suspensions such as drops. For example, for ophthalmic administration, the active ingredient may be presented as aqueous eye drops, for example, in the form of a 0.1-1.0% solution.

Formulations suitable for administration to the nose or buccal cavity include powder and self-propelling spray formulations such as aerosols and atomisers. The formulations, when dispersed, preferably have a particle size in the range 0.1 to 200μm.

A particularly desirable formulation of the present invention for use in the prophylaxis or treatment of airway smooth muscle constriction due to any cause, for example, asthma or bronchitis, is one suitable for pulmonary administration via the buccal cavity. Preferably the formulation is such that particles having a diameter of from 0.5 to 7μm, most preferably from 1 to 6μ which contain active ingredient are delivered directly into the lungs of a patient. Such formulations are conveniently in the form of dry powders for administration from a powder inhalation device or a self-propelling powder-dispensing container, for example, a self-propelling aerosol formulation in a sealed container. Preferably the powders comprise particles containing active ingredient of which at least 98% by weight have a diameter of greater than 0.5μm and at least 95% by number have a diameter of less than 7μm. Most preferably, at least 95% by weight of the particles have a diameter of greater than 1μm and at least 90% by number have a diameter of less than 6μm.

Formulations according to the invention in the form of a dry powder preferably include a solid fine powder diluent such as sugar and are typically presented in a pierceable capsule made, for example, of gelatin.

Self-propelling formulations according to the invention may be either powder-dispensing formulations or formulations dispensing the active ingredient in the form of droplets of a solution or suspension. Self-propelling powder-dispensing formulations typically comprise a liquid propellant having a boiling point of less than 18°C at atmospheric pressure. Generally, the propellant constitutes from 50 to 99.9% w/w of the composition and the active ingredient constitutes from 0.1 to 20% w/w, for example, about 2% w/w. The carrier in such formulations may include other constituents, for example, a liquid non-ionic or solid anionic surfactant, or a solid diluent (preferably having a particle size of the same order as the particles of active ingredient), or both. The surfactant generally constitutes from 0.01 to 20% w/w of the formulation, though preferably less than 1% w/w.

Self-propelling formulations of the invention wherein the active ingredient is present in solution typically comprise an active ingredient, propellant, co-solvent(s), and, advantageously, an antioxidant stabiliser. The co-solvent(s) generally constitutes from 5 to 40% w/w of the formulation, though preferably less than 20% w/w.

Formulations according to the invention may also be in the form of an optionally sterile aqueous or dilute alcoholic solution of the active ingredient for use in a nebuliser or atomiser, wherein an accelerated air stream is used to produce a fine mist consisting of small droplets of the solution. Such formulations typically contain a flavouring agent, such as saccharin sodium, and a volatile oil. A buffering agent and a surface active agent may also be incorporated in such a formulation, which may further contain a preservative such as methyl hydroxybenzoate.

Other formulations of the invention suitable for nasal administration include a coarse powder having a particle size of from 20 to 500μm which is administered in the manner in which snuff is taken, i.e. by rapid inhalation through the nasal passage from a container of the powder held close to the nose.

In addition to the aforementioned ingredients, the formulations of the invention may include one or more additional ingredients such as diluents, buffers, flavouring agents, binders, surface active agents, thickeners, lubricants, preservatives (including anti-oxidants, e.g. methyl hydroxybenzoate), emulsifying agents and the

like. Any other therapeutic ingredient may comprise one or more of an antibiotic (e.g. anti-bacterial), anti-fungal or anti-viral agent, or an anti-histamine (particularly a peripherally-acting anti-histamine). However, according to another aspect of the invention, when such other agent(s) is also present, the compound of formula (II), or a physiologically acceptable salt thereof, and the other agent(s) need not necessarily be present as a pharmaceutical formulation as hereinbefore defined, but may merely be in combination or intimate admixture, i.e. a pharmaceutically acceptable carrier need not be present.

The combination with an anti-histamine is particularly favoured for anti-asthmatic use. Such an anti-histamine may be selected from any compound described in European Patent Applications Nos. 859959 A and 117302 A. The amount and dosage regime for such an anti-histamine may be chosen from any of those recited in these two patents. Especially preferred are the anti-histamines (E)-3-(6-(3-pyrrolidino-1-(4-tolyl)prop-1E-enyl-(2-pyridyl)acrylic acid and (E)-3-(6-(3-pyrrolidino-1-(4-tolyl)prop-1E-enyl-(2-pyridyl)propionic acid. Another preferred anti-histamine is (E)-1-(4-methylphenyl)-1-(2-pyridyl)-3-pyrrolidinoprop-1-ene, otherwise known as triprolidine.

For delaying senescence of cut or picked matter, or for controlling plant growth, the compound of formula (I) and its salts are preferably presented in a suitable formulation, optionally containing one or more other agents for enhancing the freshness of the plants. Such compositions include solutions and suspensions of the compound in a suitable medium such as an aqueous medium.

The formulations of the invention may be applied by immersing part (e.g. the cut end) or all of the cut or picked plant in the formulation, by spraying the plant with the formulation before or after cutting or picking, or by applying the formulation to the root structure before or after cutting or picking. Thus the formulation may be applied to the root structure while the plant is still in the ground by spreading the formulation on the soil around the plant from where it is conveyed to the roots by rainwater or other watering means. When applied in aqueous solution, the compounds of the invention may be presented in a concentration of from 1μM to 1M, for example, from 100μM to 100mM. A typical concentration is about 1mM.

The compound of the invention and its salts may be prepared by bis-acetylation of (E)-N-{1(S)-methyl-3-[3-(4-fluorophenoxy)phenyl]prop-2-en-1-yl}hydroxylamine using, for example, acetyl chloride, in the presence of a base, such as pyridine, in an aprotic solvent, followed by de-O-acetylation using, for example, anhy. $K_2CO_3$ in an alcohol.

(E)-N-{1(S)-Methyl-3-[3-(4-fluorophenoxy)phenyl]prop-2-en-1-yl}hydroxylamine may be prepared by deprotecting a compound of formula (II)

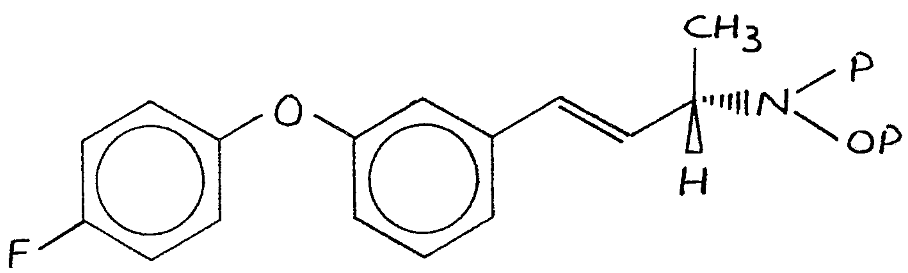

(II)

wherein P is a protecting group, such as t-butoxycarbonyl, using, for example, trifluoroacetic acid in a aprotic solvent.

The compound of formula (II) may be prepared by

(a) reacting (E)-3-[1(R)-methyl-3-(4-fluorophenoxy)phenyl]prop-2-en-1-ol with a suitably protected form of hydroxylamine, such as N,O-bis(t-butoxycarbonyl)hydroxylamine, in the presence of, for example, diethylazidocarboxylate and triphenylphosphine in an aprotic solvent; or

(b) reacting a compound of formula (III)

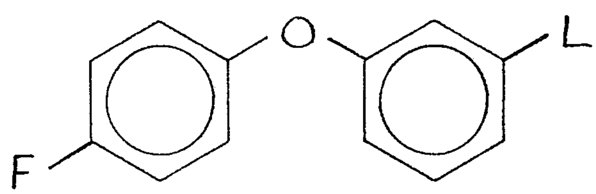

(III)

wherein L is a leaving group more labile than fluoro, such as bromo, with a compound of formula (IV)

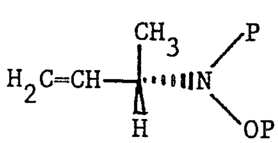

(IV)

wherein P is as hereinbefore defined, in a polar solvent in the presence of base, for example, anhy.

$K_2CO_3$/DMF, or using palladium(II) acetate and tri-o-tolylphosphine in a basic solvent, such as triethylamine, at an elevated temperature.

(E)-3-[1(R)-Methyl-3-(4-fluorophenoxy)phenyl]prop-2-en-1-ol may be prepared by stereospecifically reducing (E)-1-[3-(4-fluorophenoxy)phenyl]but-1-en-3-one using, for example, a suitable oxazaborolidine, such as that derived from (S)-2-(diphenylhydroxymethyl)pyrrolidine and $BH_3$.THF or methylboronic acid, in an aprotic solvent, or any other suitable chiral reducing agent known from the literature, for example, a lithium aluminium hydride complex modified by a chiral ligand such as binaphthyl.

(E)-1-[3-(4-Fluorophenoxy)phenyl]but-1-en-3-one may be prepared by reacting 3-(4-fluorophenoxy)benzaldehyde with a suitable Wittig reagent, such as dimethylacetylmethylphosphonate, in the presence of base, for example, anhy. $K_2CO_3$/THF or NaOH/acetone.

Compounds of formula (III) may be prepared by reacting a Group I 4-fluorophenolate, typically the potassium salt, with a compound of formula (V)

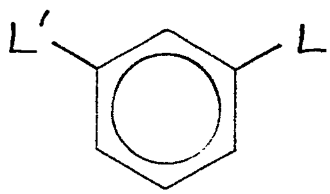

(V)

wherein L′ is a leaving group more labile that L, such as fluoro when L is bromo, in a suitable solvent such as 1-methylpyrrolidin-2-one.

Compounds of formula (IV) may be prepared by partially reducing a compound of formula (VI)

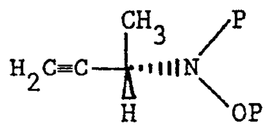

(VI)

wherein P is as hereinbefore defined, for example, by catalytic hydrogenation using, for example, Pd/$BaSO_4$ in pyridine.

Compounds of formula (VI) may be prepared by reacting a compound of formula (VII)

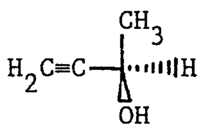

(VII)

with a suitably protected form of hydroxylamine, such as N,O-bis(t-butoxy-carbonyl)hydroxylamine, in the presence of, for example, diethylazidocarboxylate and triphenylphosphine in an aprotic solvent.

3-(4-Fluorophenoxy)benzaldehyde, Group I 4-fluorophenolates and compounds of formulae (V) and (VII) are commercially available or may be prepared by methods known to a skilled man or available from the chemical literature.

Alternatively, the compound of formula (I) may be obtained by preparing the racemic product and isolating therefrom the desired enantiomer, for example, using a chiral chromatography column or by preparing and separating suitable diastereoisomers. The racemic product may be prepared by synthetic routes analogous to those described above wherein the compound of formula (II) is racemic or by the route described in European Patent Application No. 88306446 (Synthetic Example 4).

Optimal conversion of the compound of formula (I) to a corresponding salt may conveniently be effected by reaction with an appropriate organic or mineral acid, or with a base.

For a better understanding of the invention, the following Example is given by way of illustration.

Synthetic Example

Preparation of (E)-N-{1(S)-methyl-3-[3-(4-fluorophenoxy)phenyl]prop-2-en-1-yl}acetohydroxamic acid

(a) 1-Bromo-3-(4-fluorophenoxy)benzene

A solution of KOH (10.7g) in water (30ml) was added to a stirred solution of 4-fluorophenol (21.4g, Aldrich) in methanol (200ml). After addition was complete, the mixture was evaporated in vacuo and the residual solid ground to a powder.

3-Fluorobromobenzene (33.4g, Fluorochem) was added to a stirred suspension of the ground solid in 1-methylpyrrolidin-2-one. After addition was complete, the mixture was refluxed under $N_2$ for 24 hours. The cooled mixture was poured into water (1000ml), extracted with ether (3 x 200ml) and the combined extracts washed with 1N aqu. NaOH (2 x 200ml), 2N aqu. HCl (200ml) and water (200ml), dried over $Na_2SO_4$ and

evaporated in vacuo to give a dark oil. The latter was distilled to give the desired product as a pale yellow oil (50.9g), bp 86-94°C/0.25mm Hg.

(b) N,O-Bis(t-butoxycarbonyl)but-3-yn-2(S)-ol

Diethylazidocarboxylate (117g) was added under $N_2$ at -78°C to a stirred mixture of but-3-yn-2(R)-ol in ether (5.0M, 100ml, prepared by the method described in J. Med. Chem. 31, 1563 (1988)) and N,O-bis(t-butoxycarbonyl)hydroxylamine (110g) in toluene (1000ml). After addition was complete, the mixture was warmed to room temperature over 2 hours. 40-60 pet.ether (1000ml) was added and the mixture filtered through silica. The filtrate was evaporated in vacuo and the residue then taken up in $CHCl_3$ (1000ml), copper(I) chloride (50g) added and the mixture stirred for 3 hours. The mixture was evaporated in vacuo and the residue taken up in ether (1000ml), then filtered and the filtrate evaporated in vacuo to give a pale yellow oil (122g) which crystallised from 40-60 pet. ether to give the desired product as colourless prisms (80g), mp 83-84°C, $[\alpha]_D^{23}$ -22.6° (c = 2.0, $CHCl_3$).

(c) N,O-Bis(t-butoxycarbonyl)but-3-en-2(S)-ol

The product from step (b) (14.3g) was taken up in pyridine (100ml) and hydrogenated at atmospheric pressure and room temperature in the presence of 5% palladium/barium sulphate (1.5g). Hydrogen uptake 1100ml. The mixture was evaporated in vacuo and the residue taken up in ether (250ml), washed with 10% w/v aqu. citric acid (2 x 250ml) and satd. aqu. NaCl (250ml), dried over $MgSO_4$ and evaporated in vacuo to give the desired product as a pale yellow oil (12.9g).

(d) (E)-N,O-Bis(t-butoxycarbonyl)-N-{1(S)-methyl-3-[3-(4-fluorophenoxy)phenyl]prop-2-en-1-yl}hydroxylamine

Palladium(II) acetate (0.57g) was added to a solution of the product from step (a) (13.4g), the product from step (c) (12.9g) and tri-o-tolylphosphine (1.54g) in triethylamine (50ml) in a glass pressure vessel. The mixture was stirred at 140°C for 6 hours, then cooled, ether (200ml) added and filtered through celite. The filtrate was evaporated in vacuo and the residue taken up in ether (400ml), washed with 10% w/v aqu. citric acid (400ml) and satd. aqu. NaCl (200ml), dried over $MgSO_4$/charcoal and evaporated in vacuo to give the desired product as a yellow oil which was used without further purification.

(e) (E)-N-{1(S)-Methyl-3-[3-(4-fluorophenoxy)phenyl]prop-2-en-1-yl}acetohydroxamic acid

Trifluoroacetic acid (50ml) was added under $N_2$ at 0°C to a solution of the product from step (d) in DCM (100ml). The mixture was allowed to warm to room temperature over 3 hours, then evaporated in vacuo and the residue taken up in satd. aqu. $NaHCO_3$ (200ml) and ether (250ml). The ether phase was separated, washed with satd. aqu. NaCl (200ml), dried over $MgSO_4$ and evaporated in vacuo.

The hydroxylamine residue was taken up in DCM (250ml) and pyridine (50ml) and N,N-dimethylaminopyridine (0.5g) was added at 0° C followed by the addition of a solution of acetyl chloride (12ml) in DCM (20ml). After 2 hours, ether (250ml) was added and the mixture washed with 2N aqu. HCl (2 x 250ml), satd. aqu. $NaHCO_3$ (250ml) and satd. aqu. NaCl (200ml), dried over $MgSO_4$ and evaporated in vacuo. The residue was flash chromatographed through a silica column using DCM as eluant to give the bis-acetyl compound (7.0g).

The bis-acetyl compound was taken up in methanol (150ml) and stirred in the presence of anhy. $K_2CO_3$ (2.0g) for 1 hour. The mixture was evaporated in vacuo and the residue taken up in ether (200ml) and 3N aqu. NaOH (400ml). The aqueous phase was separated, washed with ether/40-60 pet.ether (1:1 v/v), acidified to pH 4 with 5N aqu. HCl, and extracted with ether (400ml). The extract was washed with satd. aqu. NaCl (200ml), dried over $MgSO_4$ and evaporated in vacuo to give the desired product as a light tan oil (4.0g), $[\alpha]_D^{23}$ - 119.2° (c = 1.25, EtOH). 200MHz $^1H$ NMR and MS consistent with proposed structure.

Pharmaceutical Formulation Examples

The following examples serve only to illustrate suitable formulations of the present invention and should in no way be construed as limitations thereof. The "active ingredient" in the formulations may be any compound or physiologically acceptable salt thereof in accordance with the invention.

Example A: Tablet

| | Per tablet |
|---|---|
| Active Ingredient | 5.0 mg |
| Lactose | 82.0 mg |
| Starch | 10.0 mg |
| Povidone | 2.0 mg |
| Magnesium Stearate | 1.0 mg |

Mix together the active ingredient, lactose and starch. Granulate the powders using a solution of povidone in purified water. Dry the granules, add the magnesium stearate and compress to produce tablets (100mg per

tablet).

Example B: Ointment

| | | |
|---|---|---|
| Active Ingredient | | 1.0 g |
| White Soft Paraffin | to | 100.0 g |

Disperse the active ingredient in a small volume of the vehicle. Gradually incorporate this into the bulk to produce a smooth, homogeneous product. Fill into collapsible metal tubes.

Example C: Cream for topical use

| | | |
|---|---|---|
| Active Ingredient | | 1.0 g |
| Polawax GP 200 | | 20.0 g |
| Lanolin Anhydrous | | 2.0 g |
| White Beeswax | | 2.5 g |
| Methyl hydroxybenzoate | | 0.1 g |
| Distilled Water | to | 100.0 g |

Heat the Polawax, beeswax and lanolin together at 60°C. Add a solution of methyl hydroxybenzoate. Homogenise using high speed stirring. Allow the temperature to fall to 50°C. Add and disperse the active ingredient. Allow to cool with slow speed stirring.

Example D: Lotion for topical use

| | | |
|---|---|---|
| Active Ingredient | | 1.0 g |
| Sorbitan Monolaurate | | 0.6 g |
| Polysorbate 20 | | 0.6 g |
| Cetostearyl Alcohol | | 1.2 g |
| Glycerin | | 6.0 g |
| Methyl Hydroxybenzoate | | 0.2 g |
| Purified Water B.P. | to | 100.00 ml |

The methyl hydroxybenzoate and glycerin were dissolved in 70ml of the water at 75°C. The sorbitan monolaurate, Polysorbate 20 and cetostearyl alcohol were melted together at 75°C and added to the aqueous solution. The resulting emulsion was homogenised, allowed to cool with continuous stirring and the active ingredient added as a suspension in the remaining water. The whole was stirred until homogeneous.

Example E: Eye drops

| | | |
|---|---|---|
| Active Ingredient | | 0.5 g |
| Methyl Hydroxybenzoate | | 0.01 g |
| Propyl Hydroxybenzoate | | 0.04 g |
| Purified Water B.P. | to | 100.00 ml |

The methyl and propyl hydroxybenzoates were dissolved in 70ml of purified water at 75°C and the resulting solution allowed to cool. The active ingredient was then added and the solution made up to 100ml with purified water. The solution was sterilised by filtration through a membrane filter of 0.22μm pore size and packed aseptically into suitable sterile containers.

Example F: Injectable solution

| | | |
|---|---|---|
| Active Ingredient | | 10.0 mg |
| Water for Injections B.P. | to | 1.0 ml |

The active ingredient was dissolved in half of the Water for Injections and then made up to volume and sterilised by filtration. The resulting solution was distributed into ampoules under aseptic conditions.

Pulmonary formulations

In Examples G and H below, the "active ingredient" is the compound of the Synthetic Example or a physiologically acceptable salt thereof.

Example G: Powder capsules for inhalation

| | |
|---|---|
| Active Ingredient (0.5-7.0µm powder) | 4 mg |
| Lactose (30-90µm powder) | 46.0 mg |

The powders were mixed until homogeneous and filled into suitably sized hard gelatin capsules (50mg per capsule).

Example H: Inhalation aerosol

| | | |
|---|---|---|
| Active Ingredient (0.5-7.0µm powder) | | 200 mg |
| Sorbitan Trioleate | | 100 mg |
| Saccharin Sodium (0.5-7.0µm powder) | | 5 mg |
| Methanol | | 2 mg |
| Trichlorofluoromethane | | 4.2 mg |
| Dichlorodifluoromethane | to | 10.0 ml |

The sorbitan trioleate and menthol were dissolved in the trichlorofluoromethane. The saccharin sodium and active ingredient were dispersed in the mixture which was then transferred to a suitable aerosol canister and the dichlorofluoromethane injected through the valve system. This composition provides 2mg of active ingredient in each 100µl dose.

Biological Assay

In vitro inhibition of 5-lipoxygenase (5-LO) and cyclo-oxygenase (CO)

Blood from normal aspirin-free volunteers was centrifuged to separate leukocytes from red cells and platelets. The leukocytes were homogenised and 5µM arachidonic acid added, followed by incubation at 37°C for 5 minutes. The reaction was stopped by boiling and radioimmunoassays conducted for leucotriene $B_4$ and thromboxane $B_2$, products derived from 5-lipoxygenase and cyclo-oxygenase respectively. Results were calculated as $IC_{50}$(µM) activity against each enzyme.

| | LO | CO |
|---|---|---|
| $IC_{50}$(µM) activity | 0.15 | 3.0 |

**Claims**

1. (E)-N-{1(S)-methyl-3-[3-(4-fluorophenoxy)phenyl]prop-2-en-1-yl} acetohydroxamic acid and salts thereof.

2. A pharmaceutical formulation comprising (E)-N-{1(S)-methyl-3-[3-(4-fluorophenoxy)phenyl]prop-2-en-1-yl}acetohydroxamic acid or a physiologically acceptable salt thereof, a pharmaceutically acceptable carrier and/or excipient and, optionally, one or more other pharmacologically active agents.

3. A formulation according to claim 2, wherein the formulation is adapted for pulmonary administration.

4. A process for the preparation of (E)-N-{1(S)-methyl-3-[3-(4-fluorophenoxy)phenyl]prop-2-en-1-yl}acetohydroxamic acid and salts thereof, which comprises the de-O-acetylation of (E)-N,O-bis(acetyl)-N-{1(S)-methyl-3-[3-(4-fluorophenoxy)phenyl]prop-2-en-1-yl}-hydroxylamine followed, optionally, by conversion of the product to a corresponding salt.

5. A process according to claim 4, wherein the (E)-N,O-bis(acetyl)-N-{1(S)-methyl-3-[3-(4-fluorophenoxy)phenyl]prop-2-en-1-yl} hydroxylamine is prepared by the bis-acetylation of (E)-N-{1(S)-methyl--3-[3-(4-fluorophenoxy)phenyl]prop-2-en-1-yl} hydroxylamine.

6. A process according to claim 5, wherein the (E)-N-{1(S)-methyl-3-[3-(4-fluorophenoxy)phenyl]prop-2-en-1-yl}hydroxylamine is prepared by the deprotection of (E)-N,O-bis(t-butoxycarbonyl)-N-{1(S)-methyl-3-[3-(4-fluorophenoxy)phenyl]prop-2-en-1-yl}hydroxylamine.

7. A process according to claim 6, wherein the (E)-N,O-bis(t-butoxycarbonyl)-N-{1(S)-methyl-3-[3-(4-fluorophenoxy)phenyl]prop- 2-en-1-yl}hydroxylamine is prepared

(a) by reacting (E)-3-[1(R)-methyl-3-(4-fluorophenoxy)-phenyl]prop-2-en-1-ol with N,O-bis(t-butoxycarbonyl)-hydroxylamine; or

(b) by reacting 1-bromo-3-(4-fluorophenoxy)benzene with N,O-bis(t-butoxycarbonyl)but-3-en-2(S)-ol.

8. A method of treatment of a disease condition which comprises the administration of an effective amount of (E)-N-{1(S)-methyl-3-[3-(4-fluorophenoxy)phenyl]prop-2-en-1-yl}acetohydroxamic acid or of a physiologically acceptable salt thereof.

9. A method according to claim 8, wherein the disease condition is spasmogenic, allergic, pyretic, or inflammatory, involves blood platelet aggregation, or is a condition for which an analgesic is indicated.

10. A method according to claim 9, wherein the spasmogenic or allergic condition is asthma.

European Patent Office

# PARTIAL EUROPEAN SEARCH REPORT

which under Rule 45 of the European Patent Convention shall be considered, for the purposes of subsequent proceedings, as the European search report

Application number: EP 89307096.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,P, X | EP - A1 - 0 299 761 THE WELLCOME FOUNDATION) * Page 4, lines 37-39; examples 4,G,H; page 17, lines 22,33; page 18, line 10; page 19, line 9; claims 7,8 * -- | 1-4 | C 07 C 259/06<br>A 61 K 31/16 |
| D,A | EP - A2 - 0 196 184 (THE WELLCOME FOUNDATION) * Claims 5-9,11-13; column 11, line 25 * ---- | 1-4 | |

TECHNICAL FIELDS SEARCHED (Int. Cl. 5)

C 07 C 295/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7
Claims searched incompletely: -
Claims not searched: 8-10
Reason for the limitation of the search:

(Method for treatment of the human of animal body by therapy, Article 52(4) EPC)

| Place of search | Date of completion of the search | Examiner |
|---|---|---|
| VIENNA | 02-10-1989 | KÖRBER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document